# EUROPEAN PATENT APPLICATION

(11) **EP 4 480 531 A1**
(43) Date of publication of application: **25.12.2024**
(21) Application number: 24183127.0
(22) Date of filing: 19.06.2024
(51) Int. Cl.: A61N 1/378, H02J 50/10, H02J 50/90, A61N 1/372, H02J 50/80

(54) **A METHOD FOR POSITIONING AN ACCESSORY DEVICE IN RELATION TO AN INTERACTION INTERFACE OF AN ELECTRONIC IMPLANT, AND A RELATED POSITIONING DEVICE**

(30) Priority: 20.06.2023 LU 504541
(71) Applicant: PRECISIS GmbH, 69124 Heidelberg (DE)
(72) Inventor: REMMERT, Gregor, 69115 Heidelberg (DE)
(74) Representative: Aera A/S

(57) **Abstract**

A positioning device is disclosed, for positioning an accessory device in relation to an interaction interface of an electronic implant. The positioning device is configured to be worn by a bearer of the electronic implant. The positioning device comprises a base, one or more engagement interface(s), one or more drive unit(s), the accessory device, and one or more controller for controlling the one or more drive unit(s). The one or more engagement interfaces comprising a first engagement interface movably arranged in relation to the base. The accessory device comprises an interaction interface for interacting with the electronic implant. The accessory device is arranged on the first engagement interface. The positioning device is configured to move the accessory device in a curved motion. The positioning device is configured to obtain an interaction parameter indicative of a quality of an interaction between the accessory device and the electronic implant. The positioning device is configured to position, based on the obtained interaction parameter, the accessory device in relation to the base.

## Description

The present disclosure pertains to the field of electronic implants, such as implants for neurological stimulation or cardiac stimulation. The present disclosure relates to methods for positioning an accessory device with an interaction interface of an electronic implant, and a related positioning device.

### BACKGROUND

Electronic implants, such as neural implants or cardiac implants, are electronic devices that connect to organs of a biological subject to establish a biomedical prosthesis circumventing areas in the organ that have become dysfunctional. The electronic implants may be implanted underneath the skin of the biological subject. Cardiac implants, such as pacemakers, are electronic devices that connect directly to a heart of the biological subject. Neural implants, also referred to as brain implants, are electronic devices configured to stimulate the brain, such as neuronal brain tissue, of the biological subject. Neural implants electrically stimulate, block and/or record signals from single neurons or groups of neurons, such as biological neural networks, in the brain. Depending on the location of the dysfunctional area of the organ, such as an epileptic focus or a seizure focus on the brain, the surgical position of the electronic implant may vary.

To ensure a proper function of the electronic implant, an accessory device may be used for interacting with the electronic implant, such as for communicating with or charging the electronic implant. The quality of the interaction between the electronic implant and the accessory device can be heavily dependent on the relative position and/or orientation of the electronic implant and the accessory device. However, a correct positioning and orientation of the accessory device in relation to the electronic implant can be hard to achieve by a user due to the non-visible position of the electronic implant under the skin of the biological subject. US2012119699A1 describes a handheld charging coil holder that the user holds to the approximate position of his/her body where the electronic implant is located. US2018361165A1 describes a piece of garment with pockets that will hold a charging coil at a position of an electronic implant. US2018229045A1 describes a holding device for a charging coil, where the user can move the charging coil inside the holding device. The quality of communication may also be affected over time, since the bearer of the implant may get tired of holding the accessory device in position and/or may involuntarily move the accessory device out of position. An incorrect positioning and/or orientation may thus lead to a poor communication, or bad coupling between charge coils of the accessory device and the implant which may lead to an extended charging time.

### SUMMARY

Accordingly, there is a need for devices and methods for positioning an accessory device in relation to an interaction interface of an electronic implant, which may mitigate, alleviate, or address the shortcomings existing and may provide an improved quality of interaction between the electronic implant and the accessory device.

A positioning device is disclosed, for positioning an accessory device in relation to an interaction interface of an electronic implant. The positioning device may be configured to be worn by a bearer of the electronic implant. The positioning device comprises a base, one or more engagement interface(s), one or more drive unit(s), the accessory device, and one or more controller for controlling the one or more drive unit(s). The one or more engagement interfaces comprising a first engagement interface movably arranged in relation to the base. The accessory device comprises an interaction interface for interacting with the electronic implant. The accessory device is arranged on the first engagement interface. The positioning device may be configured to move the accessory device in a curved motion. The positioning device is configured to obtain an interaction parameter indicative of a quality of an interaction between the accessory device and the electronic implant. The positioning device is configured to position, based on the obtained interaction parameter, the accessory device in relation to the base.

Further, a method is disclosed, performed in a positioning device, for positioning an accessory device in relation to an interaction interface of an electronic implant. The positioning device comprises a base, one or more engagement interfaces, one or more drive unit(s), the accessory device, and one or more controller for controlling the one or more drive unit(s). The one or more engagement interface(s) are movably arranged in a translatory manner in relation to the base by means of the one or more drive unit(s). The accessory device comprises an interaction interface for interacting with the electronic implant. The accessory device is arranged on one of the one or more positioning members. The method comprises obtaining an interaction parameter indicative of a quality of an interaction between the accessory device and the electronic implant. The method may comprise moving the accessory device in a curved motion. The method comprises positioning, based on the obtained interaction parameter, the accessory device in relation to the base.

It is an advantage of the present disclosure that the positioning device moves the accessory device between positions in a curved motion, such as along a curved trajectory following a contour of a body part of the bearer wherein the implant is located, such as under the skin of the scalp or chest. As the contour of most body parts is curved, a housing or base fitting the contour, or engagement interfaces thereof, will generally also have a curved shape. The curved motion enables the positioning device to keep an at least substantially constant distance between the accessory device and the body part during movement. This is advantageous since the obtained interaction parameters will typically depend on the distance between the accessory device and the electronic implant at the time of obtaining the interaction parameters. If the accessory device were to perform straight, i.e. tangential, movement relative to the contour of the body part, the distance to an electronic implant magnitude could increase even if the accessory device were moving in its general direction. It is also an advantage of the present disclosure that the positioning device moves the accessory device between positions that all have at least substantially the same distance to the body part of the bearer.

It is an advantage of the present disclosure that the positioning device can determine, based on the obtained interaction parameters, a position of the accessory device that optimizes the interaction, such as a charging and/or communication, between the accessory device and the electronic implant. Thereby, the accessory device can be positioned in relation to the electronic implant, so that an interaction interface, such as a charging interface and/or a communication interface, of the accessory device overlaps with an interaction interface of the electronic implant without relying on magnets in the electronic implant for attracting and securing the accessory device to the electronic implant. By aligning the accessory device so that the interaction interface of the accessory device overlaps with an interaction interface of the electronic implant, the quality of the interaction between the accessory device and the electronic implant can be improved. Another benefit is that the position of the accessory device can be corrected by the positioning device in case its relative position has accidentally changed, for example due to movements of the bearer of the implant. Since the positioning and securing of the accessory device is done entirely by the positioning device, the electronic implant does not require to be equipped with a magnet for attracting and positioning the accessory device. The positioning device thus enables a magnet-free electronic implant. By making the electronic implant magnet-free, such as removing the magnet from the electronic implant, the negative influence on other diagnostics processes, such as Magnetic Resonance Imaging (MRI), can be reduced. Ferromagnetic parts, such as magnets are known to create artifacts, such as distortions of the image, in the MRI that can affect the quality of the MRI exam. Typically, permanent magnets may cause complete image deletion in the MRI due to high susceptibility changes, such as high changes to the extent the magnet is magnetized in an external magnetic field of the MRI scanner. Furthermore, since the positioning device enables a removal of magnets in the electronic implant, no intervention, such as removal of the electronic implant, is required before performing the MRI scan.

### BRIEF DESCRIPTION OF THE DRAWINGS

The above and other features and advantages of the present disclosure will become readily apparent to those skilled in the art by the following detailed description of examples thereof with reference to the attached drawings, in which:
Fig. 1 illustrates a first example positioning device according to one or more examples of this disclosure,
Fig. 2 illustrates a second example positioning device according to one or more examples of this disclosure,
Fig. 3 illustrates a third example positioning device according to one or more examples of this disclosure,
Figs. 4A-4B illustrate perspective views of an accessory device of the positioning device of Fig. 3 seen from a base facing side and a bearer facing side of the accessory device,
Figs. 5A-5C illustrate cross section views of the example positioning device illustrated in Figs. 3-4B according to this disclosure.
Figs. 6A-6B illustrate example bases of the positioning device according to one or more examples of the current disclosure,
Fig. 7 is a flow-chart illustrating an example method, performed in a positioning device, for positioning an accessory device in relation to an interaction interface of an electronic implant according to this disclosure, and
Fig. 8 is a block diagram illustrating an example positioning device according to this disclosure.

### DETAILED DESCRIPTION

Various examples and details are described hereinafter, with reference to the figures when relevant. It should be noted that the figures may or may not be drawn to scale and that elements of similar structures or functions are represented by like reference numerals throughout the figures. It should also be noted that the figures are only intended to facilitate the description of the examples. They are not intended as an exhaustive description of the disclosure or as a limitation on the scope of the disclosure. In addition, an illustrated example needs not have all the aspects or advantages shown. An aspect or an advantage described in conjunction with a particular example is not necessarily limited to that example and can be practiced in any other examples even if not so illustrated, or if not so explicitly described.

The figures are schematic and simplified for clarity, and they merely show details which aid understanding the disclosure, while other details have been left out. Throughout, the same reference numerals are used for identical or corresponding parts.

Electronic implants, such as rechargeable electronic implants, can be surgically positioned at different positions under the skin of a biological subject, such as a person. The position of the electronic implant may be selected based on the area to be treated, such as based on an epileptic focus of the biological subject. In order to enable optimal interaction between an external accessory device, such as charging by an external charging unit, a positioning and, if necessary, an orientation of the accessory device in relation to an interaction interface of the electronic implant is relevant for the quality of the interaction between the accessory device and the electronic implant. For example, the positioning and/or the orientation of an external charging device in relation to a charging coil in the electronic implant is relevant for the quality of the coupling and thus for the quality of the energy transfer between the external charging device and the charging coil. The quality of the charging also affects the charging time.

To enable an inductive energy coupling between the charging device and the electronic implant, the charging device typically has to be fixed in a correct place by a user, such as a bearer of the electronic implant. There are various techniques for influencing the location and/or position of the charging device and the receiving unit, such as the charging coil of the electronic implant, in relation to one another.

Some systems, such as cochlear implants, may use permanent magnets to fixate the position of the accessory device to the implant, without having to use mechanical fixation means, such as hoods, bands and/or straps. Permanent magnets however have the disadvantage that ferromagnetic material and magnets lead to considerable artifacts in the imaging during an MRI acquisition which negatively affects the imaging.

Since the position of electronic implants can vary from bearer to bearer, a bearer-specific charging device is required in order to position the accessory device, such as the charging device, in the correct location.

The current disclosure provides a positioning device for positioning an accessory device in relation to an interaction interface of an electronic implant, which provides a high-quality interaction, such as communication with and/or charging of the electronic implant, without generating MRI artifacts during an MRI scan of the bearer of the implant.

A positioning device for positioning an accessory device in relation to an interaction interface of an electronic implant and a method, performed in a positioning device, for positioning the accessory device in relation to an interaction interface of an electronic implant is disclosed. Positioning the accessory device in relation to the interaction interface of the electronic implant can herein be seen as positioning and/or orientating a component of the accessory device with a corresponding component of the electronic implant, such as positioning and/or orientating a transmitting charging coil of the accessory device with a receiving charging coil of the electronic implant, and/or positioning and/or orientating a communication interface of the accessory device with a communication interface of the electronic implant.

The positioning device is configured to be worn by a bearer of the electronic implant. The positioning device comprises a base, one or more engagement interfaces, one or more drive unit(s), the accessory device, and one or more controller for controlling the one or more drive unit(s). The one or more drive units may be one or more of a motor, such as an electrical motor, a spring-loaded drive unit, or any other actuator configured to perform a movement, such as a translation, of the accessory device in relation to the base, such as along the first engagement interface and/or the second engagement interface. An engagement interface can herein be seen as an assembly configured to enable the accessory device to be movably secured to the base of the positioning device.

The accessory device comprises an interaction interface for interacting with the electronic implant. The accessory device is arranged on the first engagement interface. The positioning device is configured to obtain, such as determine and/or receive, an interaction parameter indicative of a quality of an interaction between the accessory device and the electronic implant. The interaction parameter may be one or more of a charging parameter and/or a communication parameter associated with, such as related to, the electronic implant. The communication parameter may be indicative of a signal quality, such as a bitrate, bandwidth, signal strength, such as a Received Signal Strength Indicator (RSSI), a signal to noise ratio (SNR), a Q-Factor, and/or an RSS, of the signal communicated with the electronic implant via the communication interface. In one or more positioning devices, the charging parameter is one or more of a current, a voltage, a power, and a charging quality, generated in the first wireless charging interface, such as in the inductive charging coil or the CPT coupling coupler of the electronic implant. In one or more examples, the charging parameter is indicative of an alignment of the accessory device with the electronic implant, such as an alignment between the transmitting charging coil of the accessory device and the receiving charging coil of the electronic implant. In one or more example methods, the charging parameter is indicative of a frequency bandwidth, such as a frequency bandwidth of the voltage and/or current generated in the charging coil. In one or more example methods, the charging parameter is indicative of a current amplitude, such as a current amplitude in the charging coil. In one or more example methods, the charging parameter is indicative of a phase alignment. The phase alignment can be determined based on a phase-angle measurement. A phase delay between an output voltage and a current after a DC conversion in the wireless charging coil is a measure for distance of the first and the second wireless charging coils. The positioning device, such as the accessory device, and/or the electronic implant may be configured to determine the phase alignment using a processor circuitry, such as a Digital Signal Processor (DSP) controller comprised in the positioning device, such as the accessory device, and/or the electronic implant.

The positioning device is configured to position, based on the obtained interaction parameter, the accessory device in relation to, such as relative to, the base. Upon the positioning device being worn by a bearer, such as by a user having an implant, the positioning device is configured to position, based on the obtained interaction parameter, the accessory device in relation to the implant. When the positioning device is worn by the bearer of the implant, the base is stationary to the implant and the accessory device can be moved in relation to the base and/or the implant. In one or more example positioning devices, the positioning device is configured to move the accessory device in a curved motion, such as along a contour of the body part of the bearer in which the electronic implant is positioned. In one or more example positioning devices, positioning comprises moving the accessory device in relation to the base and/or the implant via the one or more engagement interfaces.

In one or more examples herein, the interaction interface comprises a charging interface, such as a charging coil, and a communication interface, such as a communication coil. The communication coil may be a signal transceiver coil. The positioning device may obtain a parameter indicative of a correct position from the implant, for example via the communication interface. In one or more examples herein, the communication coil may be concentrically arranged with the charging coil of the accessory device and the implant respectively. In one or more examples, the communication coil in the implant may be a transmitting coil which may be configured to send a signal indicative of a position of the accessory device, such as a correct position of the accessory device, to the communication coil of the accessory device, which may be a receiver coil. In one or more examples herein, the implant and/or the accessory device may use the charging coil for both charging and for signal transmitting and/or receiving.

In one or more examples herein, the respective charging coils of the implant and the accessory device may be larger in diameter than the respective communication coils of the implant and the accessory device. In one or more examples herein, the larger charging coil may be used for coarse positioning, followed by fine tuning of the positioning by using the smaller in diameter communication coils.

In one or more examples herein, a signal indicative of the interaction parameter, such as the charging parameter and/or the communication parameter may be obtained from the electronic implant. The signal may be obtained via a communication interface of the accessory device. In one or more examples herein, the signal indicative of the interaction parameter is continuously or periodically communicated. In one or more examples herein, the signal indicative of the interaction parameter is non-continuously or non-periodically communicated. In one or more example positioning devices, the accessory device may obtain the interaction parameter by measuring the interaction parameter, such as the quality of the charging parameter and/or communication parameter, associated with the electronic implant. In one or more example positioning devices, the accessory device may obtain the interaction parameter by receiving a message comprising an indicator indicative of the interaction parameter from the electronic implant. The interaction parameters may be obtained using the interaction interface comprised in the accessory device. The quality of the charging parameter may in one or more example positioning devices be obtained as a percentage of the maximum charging quality.

The one or more engagement interfaces comprise a first engagement interface movably arranged in relation to the base, and when the positioning device is carried by a bearer, in relation to the implant. In one or more example positioning devices, the first engagement interface is configured to perform, such as enable the accessory device to perform, a first rectilinear motion in relation to the base, so that if the base and/or the first engagement interface is concave, the accessory device will perform a curved motion. In one or more example positioning devices, the first engagement interface is configured to perform, such as enable the accessory device to perform, a first curved motion in relation to the base. In one or more example positioning devices, the first engagement interface comprises a bracket and a first sliding connector for connecting the accessory device to the bracket. The bracket can be seen as an object that connects the accessory device to the base. The accessory device may be movably arranged to the bracket, via the first sliding connector. In one or more example positioning device(s), the first sliding connector may comprise an elongated slot, an elongated rail, and/or an elongated groove, arranged on the bracket. The elongated slot, rail or groove may extend in a longitudinal direction of the bracket. The elongated slot may be configured to receive a guide member of the accessory device. The guide member may be configured to movably secure the accessory device to the bracket. The bracket may be made out of plastic or metal. The accessory device may be configured to move along the first sliding connector by means of, such as using, a first drive unit of the one or more drive units. In one or more example positioning devices, the first drive unit may be a rack and pinion type drive unit. In one or more example positioning devices, the first drive unit may be a belt drive unit, such as a drive unit comprising a belt for moving the accessory device along the first engagement interface and/or the second engagement interface. In one or more example positioning devices, the first sliding connector, such as the elongated slot, elongated groove, and/or the elongated rail, may comprise the rack part of the rack and pinion type drive unit, such as a linear gear being arranged on an engagement surface for engaging the guide member of the accessory device. The accessory device, such as the guide member of the accessory device, may comprise the pinion part of the rack and pinion type drive unit, such as a circular gear configured to engage the linear gear of the rack. In one or more example methods, the guide member of the accessory device may be the pinion. The pinion may be rotatably arranged to the accessory device. In one or more example positioning devices, the pinion may be configured to be rotatably driven by an electric motor arranged in the accessory device and being controlled by the control circuitry of the positioning device.

In one or more example positioning devices, the bracket may be a solid bracket. The accessory device may comprise a bracket receiver for slidably receiving the bracket. The bracket receiver may be configured to, at least partially, surround the bracket from four sides of the bracket. The bracket receiver may comprise two L-shaped members mounted to a bracket facing surface of the accessory device at a distance corresponding to the width of the bracket. The bracket may be inserted between the two L-shaped bracket so that the bracket is secured between the bracket facing surface of the accessory device and the L-shaped members. In one or more example positioning members, the bracket receiver may be U-shaped, wherein the free ends of the U-shape are mounted to the bracket facing surface. The U-shaped bracket receiver and the bracket facing surface of the accessory device thereby create a second slot for receiving the bracket. In other words, the U-shaped bracket receiver and the bracket facing surface of the accessory device may fully surround the bracket.

The accessory device may be movably arranged in a primary direction via the first engagement interface. In one or more example positioning devices, the primary direction is along the longitudinal direction of the bracket.

In one or more example positioning devices, the positioning device is configured to move the accessory device in a curved motion, such as along a contour of the body part of the bearer in which the electronic implant is located. In one or more example positioning devices, the bracket and/or the first sliding connector, such as the elongated slot, rail, or groove, may have a curved shape. The accessory device may thus perform a curved motion when moving along the sliding connector. It is understood that in embodiments where the base is a headpiece, the curved or concave shape of the base and/or the one or more engagement interfaces and/or brackets and/or sliding connectors will be similar to, such as follow, a contour of the head of the bearer. Similarly, in embodiments where the base is configured to be positioned on the chest of the bearer, the curved or concave shape of the base and/or the one or more engagement interfaces and/or brackets and/or sliding connectors will be similar to, such as follow, a contour of the part of the chest of the bearer wherein the electronic implant is located.

In one or more example positioning devices, the positioning device comprises a second engagement interface. The first engagement interface may be movably arranged, such as in relation to the base, in a secondary direction via the second engagement interface. In one or more example positioning devices, the second engagement interface is configured to perform a second rectilinear motion in relation to the base, so that if the base and/or the second engagement interface is concave, the accessory device will perform a curved motion. In one or more example positioning devices, the second engagement interface is configured to perform a second curved motion in relation to the base The second motion is different to the first motion. In one or more example positioning devices, the bracket of the first engagement interface is movably arranged to the base via one or more second sliding connectors. The one or more second sliding connectors may comprise the bracket, such as one or more ends of the bracket, and one or more second elongated slot(s), elongated rail(s), and/or elongated groove(s), for receiving the ends of the bracket. In one or more example positioning devices, the one or more second sliding connectors may extend in a direction substantially perpendicular to the first sliding connector. In other words, the primary direction and the secondary direction may be substantially perpendicular to each other. It is generally understood that the perpendicularity of directions projected onto a curved or concave surface are only locally perpendicular, such as perpendicular in an infinitesimal region intersection of two lines. The first engagement interface, such as the bracket, may be configured to move along the one or more second sliding connector(s) by means of, such as using, one or more second drive unit(s) of the one or more drive unit(s). In one or more example positioning devices, the one or more second drive unit(s) may be a second rack and pinion type drive unit. In one or more example positioning devices, the one or more second sliding connectors, such as the one or more elongated slot(s), elongated groove(s), and/or elongated rail(s), may comprise the rack part of the rack and pinion type drive unit. The rack part may be a linear gear being arranged on an engagement surface for engaging a respective end of the bracket. The one or more end(s) of the bracket may comprise a respective pinion part of the rack and pinion type drive unit, such as a circular gear configured to engage the linear gear of a respective second sliding connector. In one or more example positioning devices, the one or more pinion(s) of the second rack and pinion type drive unit may be configured to be rotatably driven by one or more electric motor(s) arranged on the bracket, such as on the end(s) of the bracket and being controlled by the control circuitry of the positioning device.

In one or more example positioning devices, the second engagement interface is configured to perform a rotational motion in relation to the base. In one or more example positioning devices, the bracket may be movably secured to the base via a pivoting connection. The pivoting connection may be configured to enable the bracket to pivot, such as rotate, around a pivot point, such as a rotational axis, in relation to the base. The pivoting movement of the second engagement interface will inherently cause the accessory device to perform a curved motion. The pivoting movement of the second engagement interface, such as the pivoting of the bracket, may be performed by one of the one or more drive units.

In one or more example positioning devices, the accessory device is configured to be freely movable over a surface of the base. The accessory device may be configured to be sandwiched between the base and the bearer of the implant, so that the accessory device abuts the base on a first side and the bearer of the implant on an opposite second side and can move in the space between the base and the bearer. It follows that if the base has a concave shape, the accessory device will perform in a curved motion when moved by the drive units. In this case, the first engagement interface may be a set of drive rollers, such as drive wheels. The set of drive rollers may protrude from a first surface of the accessory device, the first surface of the accessory device being a base facing surface. The set of drive rollers may be connected to and may be drivable by the one or more drive units. In this example positioning device, the accessory device may further comprise one or more spacers configured to space the surfaces of the accessory device away from the base and/or from the bearer of the implant. In one or more example positioning devices, the spacers are slidably arranged on the accessory device and may be spring loaded. This allows the one or more spacers to follow the contours of the base and/or the bearer of the implant and compensate for irregularities in the contours of the base and/or the bearer.

In one or more example positioning devices, the interaction interface is a wireless charging interface, and the interaction parameter is indicative of a quality of charging. In one or more example positioning devices, the wireless charging interface is a charging coil. In one or more example positioning devices, the wireless charging interface may use an inductive, capacitive or ultrasound coupling, or combinations thereof.

In one or more example positioning devices, the accessory device is a communication device, and the interaction interface may be a communication interface. The communication interface may for example be a wireless interface for communicating with the accessory device. The interaction parameter may be indicative of a quality of communication between the accessory device and the electronic implant.

In one or more example positioning devices, the accessory device is a wireless charging device, and the interaction interface may be a wireless charging interface, such as an inductive charging coil. The inductive charging coil may for example be an inductive charging coil for wireless charging of the electronic implant. The interaction parameter may be indicative of a quality of charging between the accessory device and the electronic implant.

In one or more example positioning devices, the accessory device is both a communication device and a charging device and may comprise both a communication interface and the wireless charging interface, such as the inductive charging coil. The inductive charging coil of the electronic implant may herein be referred to as a receiving charging coil and the inductive charging coil of the accessory device may be referred to as a transmitting charging coil.

In one or more example positioning devices, the interaction interface is a wireless communication interface, and the interaction parameter is indicative of a quality of communication. The wireless communication interface is configured for wireless communications via a wireless communication system, such as via one or more of Bluetooth, Wi-Fi, Near-Field Communication (NFC), Medical Implant Communication Service (MICS), and Industrial-Scientific-Medical (ISM), or a mobile communication system, such as a 3^{rd} Generation Partnership Program (3GPP) system.

In one or more example positioning devices, the accessory device may comprise the charging interface and the communication interface.

The positioning device may be configured to perform a method for positioning the accessory device in relation to an interaction interface of an electronic implant as disclosed herein. In one or more example positioning devices, the positioning device is configured to obtain sets of interaction parameters associated with a plurality of positions of the accessory device in relation to the electronic implant. The positioning device may be configured to move the accessory device, for example using the engagement interfaces, to, such as between, the plurality of positions along a curved trajectory and obtain a set of interaction parameters at each respective position. The positioning device may be configured to determine a quality of each set of interaction parameters. The positioning device may be configured to position the accessory device in the position associated with the set of interaction parameters having the highest quality. In other words, the positioning device may, based on the obtained sets of interaction parameters, position the accessory device in a position in which charging of the electronic implant and/or communication with the electronic implant is best. The position associated with a set of interaction parameters can herein be seen as the position in which the set of interaction parameters was obtained.

In one or more example positioning devices, the positioning device is configured to obtain a first set of interaction parameters, such as a primary first set of interaction parameters, associated with a first position of the accessory device in relation to the base. The first position of the accessory device may be a current position, such as an initial position of the accessory device.

In one or more example positioning devices, the positioning device is configured to obtain a second set, such as a primary second set, of interaction parameters associated with a second position of the accessory device in relation to the base. The second position is different than the first position. The positioning device may for example be configured to move the accessory device, using the first engagement interface and/or the second engagement interface, to the second position and obtain the set of interaction parameters associated with the second position.

In one or more example positioning devices, the positioning device is configured to determine a quality of the obtained first and second set of interaction parameter.

In one or more example positioning devices, the positioning device is configured to, upon the second set of interaction parameters being indicative of a higher quality of interaction than the first set of interaction parameters, position, using one or more of the engagement interfaces, the accessory device in the second position. The quality of interaction can herein be seen as an indicator of how much of a signal transmitted via a first interaction interface that is received at a second interaction interface. The quality of interaction may be indicated as an explicit quality or implicitly using indirect measurement states, such as one or more of a current, a voltage, a power, and parameters based on a spectrum of the signal, such as a central frequency and a Q-factor of the signal, generated in the wireless charging interface.

In one or more example positioning devices, the positioning device is configured to, upon the second set of interaction parameters being indicative of a lower quality of interaction than the first set of interaction parameters, position the accessory device in the first position.

In one or more example positioning devices, the positioning device is configured to repeat the obtaining of sets of interaction parameters associated with different positions of the accessory device. Once the positioning device has moved the accessory device to the position out of the first position and the second position having the highest quality, this position may become the first position for the next repetition of the method, such as may become a secondary first position.

Upon the set of interaction parameters associated with the primary first position having a higher quality than the set of interaction parameters associated with the primary second position, the positioning device may return the accessory device to the primary first position. For the next repetition, a third position different than the primary first and the primary second position, such as a secondary second, is chosen. The secondary second position may be chosen in an opposite direction or a direction perpendicular to the direction of movement from the primary first position to the primary second position. In other words, for the new repetition the set of interaction parameters may be obtained in a direction different than the direction between the primary first position and the primary second position.

Upon the set of interaction parameters associated with the primary second position having a higher quality than the set of interaction parameters associated with the primary first position, the positioning device may keep the accessory device in the primary second position. The primary second position may then become a secondary first position for a new iteration of the method. The positioning device may be configured to move the accessory device to a secondary second position and obtain a set of interaction parameters associated with the secondary second position. The secondary second position may be located in a same trajectory, such as in a first trajectory, as the move from the primary first position to the primary second position. As long as the quality of the set of interaction parameters in the second position, such as in a tertiary second position, a quaternary second position, etc., the positioning device may continue to move the accessory device along the same trajectory until the quality of the set of interaction parameters associated with the respective second position fails to improve.

Thereafter, the positioning device may be configured to move the accessory device in a direction perpendicular to the direction of the previous trajectory, such as the first trajectory, to find an optimum position along the second trajectory, such as a position where the quality of the set of interaction parameters is optimal, such as better than the quality of the set of interaction parameters associated with other positions on a positioning area defined by the degrees of freedom of the first engagement interface and the second engagement interface.

The positioning device may thus determine whether the quality of the set of interaction parameters increases in any direction and thus determine a trajectory for moving the accessory device, in order to find an optimal position of the accessory device for interacting with the electronic implant. The optimal position can herein be seen as a position in which a quality of the set of interaction parameters are the highest, such as a position in which the set of interaction parameters don't increase in any direction upon the accessory device being moved out of the position.

In one or more example positioning devices, the first trajectory may be along an extension of the first engagement interface, such as along the extension of the first sliding connector. In one or more example positioning devices, the second trajectory may be along an extension of the second engagement interface, such as along the extension of the one or more second sliding connector(s) and/or around the pivoting connection.

In one or more example positioning devices, such as when the accessory device is configured to be freely movable over a surface of the base, the first trajectory may be any trajectory within the boundaries of the base. It follows that if the base is concave or curved to follow a contour of the head, chest, or other body part of the bearer, the trajectories over the surface of or within the boundaries of the base will be curved trajectories.

The positioning device may be configured to perform the positioning of the accessory device using a random approach, a stepwise approach and/or a gradient-based approach, or any combination thereof. In other words, the method disclosed herein for positioning the accessory device in relation to the interaction interface of an electronic implant can be performed randomly, stepwise and/or gradiently. The gradient based positioning may be based on a Gauß-Newton method.

In one or more example positioning devices, the positioning device may be configured to determine the optimal position for the accessory device using a stepwise approach. The stepwise approach may be seen as a grid-based scanning. In the stepwise approach the positioning device may be configured to compare a quality between an obtained set of interaction parameters associated with a first and a second position, such as a current position and a new position, and move the accessory device to the position associated with the set of interaction parameters having the better quality and then repeat these steps until no quality improvement can be detected.

In one or more example positioning devices, the positioning device may be configured to determine the optimal position for the accessory device using a random approach. In one or more example positioning devices, the positioning device may be configured to move over a positioning area defined by the degrees of freedom of the first engagement interface and the second engagement interface and continuously obtain sets of interaction parameters for the covered area. The movement over the positioning area may follow a random pattern or a predefined pattern. Once the positioning device has gathered the interaction parameters for the entire positioning area, the positioning device may be configured to determine a position within the positioning area having the set of interaction parameters indicative of the highest quality of interaction of the obtained sets of interaction parameters and may move the accessory device to the determined position.

In one or more example methods, the positioning device may determine the substantially optimal position using a combination of approaches. In one or more example methods, the search space is sampled using a plurality of the approaches combined. A starting point may for example be determined and the positioning device may try to find a better position based on the starting point using for example a gradient based approach, such as using a gradient descent optimization. During application of the gradient based approach, different starting points may be randomly tried in order to ensure that the gradient-based optimization is not lost in a local optimum.

The base may have a first side configured to face the bearer of the electronic implant and a second side being configured to face away from the bearer of the electronic implant. In one or more example positioning devices, the first engagement interface, the second engagement interface and the accessory device are arranged on a bearer facing side of the base. The first engagement interface and the second engagement interface may be configured to position, such as move the accessory device, within the boundaries of the base.

In one or more example positioning devices, the positioning device may be configured to seize positioning of the accessory device upon the accessory device reaching the boundary of the base. In one or more example positioning devices, the positioning device may be configured to move the accessory device to a position having the highest determined quality of the set of interaction parameters upon the accessory device reaching the boundary of the base.

In one or more example positioning devices, the positioning device may be configured to output a visual and/or acoustic signal upon the accessory device reaching the boundary of the base.

In one or more example positioning devices, the base is a mechanical device configured to support the accessory device and/or to be worn by the bearer. The base may be one or more of a headpiece, such as a hard hat (such as a helmet), spectacles, a headband, or any other device that is suitable to be worn on the head of the bearer. In one or more example positioning devices, the headpiece comprises a headband for securing the headpiece to a head of a bearer of the electronic implant, and a set of spacers for spacing the headband from the base. In one or more example positioning devices, the base is a piece of garment, configured to be worn on the body of the bearer, such as a vest, a jacket, etc.

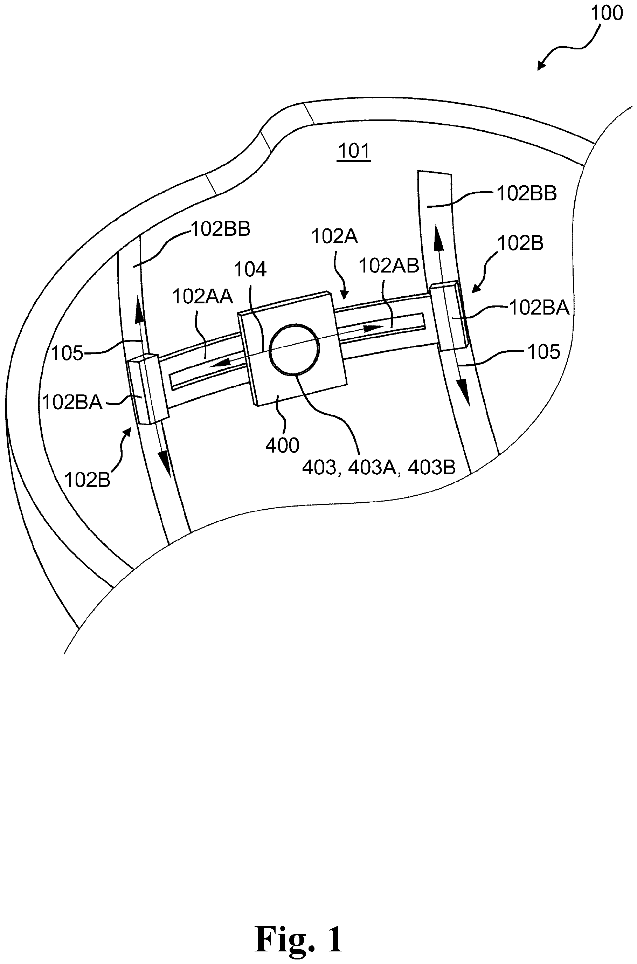

In one or more example positioning devices, the base is a housing, such as a box, in which the accessory device is movably arranged. The base, such as the housing, may thus be configured to, at least partially, enclose the engagement interfaces and the accessory device. The engagement interfaces arranged in the housing may be any of the engagement interfaces described in relation to Figs. 1-5C herein. The housing, such as the box, may have a concave surface, such as a concave outer surface, configured for facing the bearer of the implant, such as a head or a body of the bearer of the implant.

The concave surface may be configured, such as shaped, to match a contour of the bearer of the implant, such as a contour of the head and/or a trunk region of the bearer. The housing may thus be seen as being configured to be worn on the head of the bearer or on the trunk region of the bearer. The accessory device may be arranged within the housing. The housing may comprise securing means, such as straps, for securing the housing to the bearer of the implant. In one or more example methods, the first interaction interface and/or the second interaction interface are arranged within the housing. In one or more example positioning devices, the accessory device may be arranged on a movable carrier, such as a robot, enclosed in the housing, wherein the housing defines the boundaries of the movement of the movable carrier and the accessory device mounted thereto. It follows that if the housing is concave or curved to follow a contour of the head, chest, or other body part of the bearer, the movement of the movable carrier within the boundaries of the housing will be curved movement.

Further, a method is disclosed, performed in the positioning device according to the current disclosure, for positioning the accessory device in relation to an interaction interface of an electronic implant. The positioning device comprises the base, one or more engagement interfaces, one or more drive unit(s), the accessory device, and one or more controller for controlling the one or more drive unit(s). The one or more engagement interface(s) are movably arranged in a translatory manner in relation to the base, such as by means of the one or more drive unit(s). The accessory device is arranged on one of the one or more engagement interfaces.

The method comprises obtaining an interaction parameter indicative of a quality of an interaction between the accessory device and the electronic implant. The method comprises obtaining a first set of interaction parameters associated with a first position, such as a primary first position, of the accessory device, in relation to the base, wherein the first position of the accessory device is a current position, such as an initial position. The obtaining may be performed using the interaction interface, such as using the charging interface, and/or the communication interface. Obtaining the interaction parameter may comprise receiving the interaction parameter, such as from the electronic implant, or determining the interaction parameter.

The method comprises obtaining a second set of interaction parameters associated with a second position, such as a primary second position, of the accessory device in relation to the base. The second position may be different than the first position.

The method comprises positioning, based on the obtained interaction parameter, the accessory device in relation to the base. When the positioning device is in operation, such as is being used by a bearer of the electronic implant, the base is stationary in relation to the electronic implant. Hence, when positioning the accessory device in relation to the base it is also positioned in relation to the electronic implant.

In one or more example methods, the method comprises positioning the accessory device in the position being associated with the set of interaction parameters being indicative of a higher quality of interaction. In one or more example methods, positioning the accessory device comprises, upon the second set of interaction parameters being indicative of a higher quality of interaction than the first set of interaction parameters, positioning the accessory device in the second position.

In one or more example methods, positioning the accessory device comprises, upon the second set of interaction parameters being indicative of a lower quality of interaction than the first set of interaction parameters, positioning the accessory device in the first position.

The electronic implant may be a rechargeable electronic implant. The electronic implant may comprise an interaction interface, such as a charging interface and/or a communication interface, for interacting with the accessory device. The electronic implant may comprise one or more electrodes for providing electronic stimulation of tissue, such as brain tissue, of the bearer of the electronic implant. The electronic implant may be one or more of an implant for neurological applications, such as an implant for head based applications, such as an implant for brain stimulation, an implant for cortex stimulation, a cochlear implant, and/or an implant for nerve stimulation, and an implant for chest based applications, such as an implant for cardiac stimulation, such as a pacemaker, or as an implant for brain stimulation, where the Implantable Pulse Generator (IPG) is located in the chest area of the bearer of the implant. In one or more example implants, the implant may comprise a first part being head based and a second part being chest based. The head-based part may comprise one or more electrodes for stimulating brain tissue. The chest-based part may comprise an energy storage and/or the charging device.

The charging interface of the electronic implant may be a first wireless charging interface, such as a receiving charging interface or a secondary charging interface, for generating a current when the first wireless charging coil is exposed to a magnetic field or an electric field, such as a magnetic field or electric field generated by a second wireless charging interface, such as a transmitting charging interface or a primary charging interface, comprised in the accessory device. The first wireless charging interface and the second wireless charging interface may be inductive charging coils or capacitive power transfer (CPT) coupling couplers. The first wireless charging interface and the second wireless charging interface may be configured to establish a wireless energy transfer from the accessory device to the electronic implant.

The electronic implant may, in one or more examples herein, monitor a presence of the accessory device, such as may monitor one or more interaction parameters. Monitoring can herein be seen as observing over time. Monitoring the presence of the accessory device, may in one or more example methods, comprise monitoring a quality of charging, such as monitoring one or more charging parameters generated in the first wireless charging interface, such as in the inductive charging coil or the CPT coupling coupler, of the electronic implant, and/or a quality of communication, such as monitoring one or more communication parameters.

The one or more charging parameters may be one or more of a current, a voltage, a power, and parameters based on a spectrum of the signal, such as a central frequency and a Q-factor of the signal, generated in the first wireless charging interface. The Q-factor is a measure of the quality of coupling factor and can be determined by Q= w_c/(w_1+w_2), where w_c is a central frequency and w_1 and w_2 are the -3 dB frequencies of the spectrum. In one or more examples, the electronic implant may monitor the presence of the accessory device by monitoring an activation of a magnetic switch comprised in the electronic implant being activated by the presence of an external magnetic field, such as by an external magnetic field generated by the accessory device, such as by the second wireless charging interface, such as in the inductive charging coil, of the accessory device.

The electronic implant and/or the positioning device may, in one or more example positioning devices, comprise a monitoring unit, such as processor circuitry, for monitoring the interaction parameter, such as the quality of charging and/or the quality of communication. The monitoring unit, such as the processor circuitry, may be configured to monitor the quality of charging, such as the one or more charging parameters, directly on or at the charging interface, after pre-processing, and/or using digital and/or analogue monitoring solutions. A digital solution may for example be that the voltage is discretized by an Analog digital converter (ADC) and is monitored by a Micro-Controller. In the analog solution a transistor may monitor the voltage level, for example on the Base-Emitter-potential. Once a switching voltage level is reached the transistor can be enabled to switch the collector-emitter current.

In one or more examples herein, the electronic implant can communicate a signal indicative of the interaction parameter, such as the charging parameter and/or the communication parameter, to the positioning device, such as to the accessory device. The signal may be communicated upon the electronic implant detecting that the accessory device is present, such as when the positioning device has been arranged on the bearer of the electronic implant. The signal may be communicated via a communication interface of the electronic implant and/or the accessory device. The signal indicative of the charging parameter may be used by the positioning device, such as a control circuitry of the positioning device, to position the accessory device in relation to the base and the electronic implant.

In one or more exemplary embodiments, the signal is communicated to one or more of the accessory device, the bearer, or a user equipment of the bearer of the electronic implant. The user equipment may be one or more of a smart phone, a smart watch, a tablet, and a computer. In one or more examples herein, the signal indicative of the interaction parameter is continuously or periodically communicated. In one or more examples herein, the signal can be communicated upon the charging of the electronic implant starting, such as when the transmitting inductive charging coil comes close to the receiving inductive charging coil. In one or more example methods, the interaction parameter is a charging quality. The charging quality may be communicated as a percentage of a maximum charging quality.

In one or more examples herein, the signal indicative of the interaction parameter, such as the charging parameter, is communicated to the accessory device. The accessory device may be configured to communicate the quality of the interaction, such as the quality of charging, to a control circuitry of the positioning device for controlling the position of the accessory device.

In one or more examples herein, the electronic implant and/or the accessory device sends a signal indicative of the quality of interaction to a user device, such as a user device associated with the bearer of the implant or with an operator of the accessory device. The user equipment may be configured to display, for example via an application installed on the user equipment, the quality of interaction, such as the charging quality.

This allows the bearer of the electronic implant to monitor the interaction between the accessory device, such as the charging device, and the electronic implant and to manually adjust the position of the accessory device in case the quality of interaction, such as the charging quality and/or communication quality is poor. By communicating the signal to the bearer of the electronic implant, the bearer may also be informed of the charging status of the electronic implant.

Fig. 1 shows an example positioning device 100 for positioning of the accessory device 400 in relation to an interaction interface of an electronic implant according to one or more examples disclosed herein. The positioning device 100 is configured to be worn by a bearer of the electronic implant. The example positioning device 100 shown in Fig. 1 is configured to be worn on the head of a bearer. The positioning device 100 comprises a base 101. In the example shown in Fig. 1, the base 101 is a headpiece, such as a helmet.

Fig. 1 illustrates a view of a head facing side of the base 100, such as a view of an inside of the headpiece. The positioning device 100 comprises the accessory device 400 and a plurality of engagement interfaces 102, such as a first engagement interface 102A and a second engagement interface 102B. In the example positioning device 100 of Fig. 1, the first engagement interface 102A and the second engagement interface 102B, such as the first sliding connector 102AB and the one or more second sliding connectors 102BB, are arranged substantially perpendicular to each other. In other words, the first engagement interface 102A and the second engagement interface 102B are configured to move the accessory device in substantially perpendicular directions on the base 101. The accessory device 400 is arranged on the first engagement interface 102A, such as is configured to move in relation to the base 101 via the first engagement interface 102A.

The first engagement interface 102A is movably arranged in relation to the base 101. The first engagement interface 102A is configured to perform, such as enable the accessory device 400 to perform, a first rectilinear motion in relation to the base 101. The rectilinear motion can herein be seen as a motion along a line in relation to the base, so that if the base is curved, the rectilinear motion will be along a curve. The first engagement interface 102A, the second engagement interface 102B and the accessory device 400 are arranged on a bearer facing side, such as surface, of the base 101. In the example positioning device 100 of Fig. 1, the first engagement interface 102A comprises a bracket 102AA and a first sliding connector 102AB for connecting the accessory device 400 to the bracket 102AA. The bracket 102AA can be seen as an object that connects the accessory device 400 to the base 101. The accessory device 400 is movably arranged to the bracket 102AA via the first sliding connector 102AB. In the example positioning device 100 of Fig. 1, the first sliding connector 102AB comprises an elongated slot, an elongated rail, and/or an elongated groove, arranged on the bracket 102AA. The elongated slot, rail or groove may extend in a longitudinal direction of the bracket. The elongated slot or groove is configured to receive a guide member (not shown in Fig. 1) of the accessory device 400. The guide member may be configured to movably secure the accessory device 400 to the elongated slot or groove of the first sliding connector 102AB of the bracket 102AA.

The accessory device 400 may be configured to move along the first sliding connector 102A by means of, such as using, a first drive unit (not shown in Fig. 1). The first drive unit may be a rack and pinion type drive unit. The first sliding connector, such as the elongated slot, elongated groove, and/or the elongated rail, may comprise the rack part of the rack and pinion type drive unit The rack part may be a linear gear being arranged on an engagement surface of the bracket for engaging the accessory device, such as on an inside of the elongated slot of the first sliding connector 102AB or on an accessory device facing surface of the bracket 102AA. The accessory device 400, such as the guide member (not shown in Fig. 1) of the accessory device 400, may comprise the pinion part of the rack and pinion type drive unit, such as a circular gear configured to engage the linear gear on the bracket. In one or more example methods, the guide member of the accessory device may be the pinion. The pinion may be rotatably arranged to the accessory device. In one or more example positioning devices, the pinion may be configured to be rotatably driven by an electric motor arranged in the accessory device and being controlled by the control circuitry (not shown in Fig. 1) of the positioning device 100. The accessory device 400 is movable in a primary direction 104 via the first engagement interface 102A. In the example positioning device of Fig. 1, the primary direction 104 is along the longitudinal direction of the bracket 102AA.

The positioning device 100 comprises a second engagement interface 102B. The first engagement interface 102A may be movably arranged in relation to the base in a secondary direction 105 via the second engagement interface 102B. In the example positioning device of Fig. 1, the primary direction 104 is perpendicular to the longitudinal direction of the bracket 102AA. In other words, the primary direction 104 and the secondary direction 105 may be substantially perpendicular to each other. The example second engagement interface S102B of Fig. 1 is configured to perform a second motion in the secondary direction 105 in relation to the base 101. The second motion is different to the first motion. In the example positioning device 100 of Fig. 1, the bracket 102AA of the first engagement interface 102A is movably arranged to the base 101 via two second engagement interfaces S102B, such as via two second sliding connectors 102BB. The one or more second engagement interfaces S102B may comprise the bracket 102AA, such as the ends 102BA of the bracket 102AA, and the one or more sliding connectors 102BB, for receiving the ends 102BA of the bracket 102AA. The one or more second sliding connectors 102BB may be one or more of elongated rail(s), elongated groove(s), and elongated slot(s). The one or more second sliding connectors 102BB may extend in a direction substantially perpendicular to the first sliding connector 102A. The first engagement interface 102A, such as the bracket 102AA, may be configured to move along the one or more second sliding connector(s) 102BB by means of, such as using, one or more second drive unit(s) (not shown in Fig. 1). The one or more second drive unit(s) may be a second rack and pinion type drive unit. The one or more second sliding connectors 102BB, such as the one or more elongated slot(s), elongated groove(s), and/or elongated rail(s), may comprise the rack part of the rack and pinion type drive unit. The rack part may be a linear gear being arranged on an engagement surface for engaging a respective end 102BA of the bracket 102AA. The one or more end(s) 102BA of the bracket may comprise a respective pinion part of the rack and pinion type drive unit, such as a circular gear configured to engage the linear gear of the respective second sliding connector 102BB. The one or more pinion(s) of the second rack and pinion type drive unit (not shown) may be configured to be rotatably driven by one or more electric motor(s) arranged on the bracket 102AA, such as at the end(s) 102BA of the bracket 102AA and being controlled by the control circuitry of the positioning device (not shown). By controlling the movement along the first engagement interface and the second engagement interface in relation to each other, the positioning device can move the accessory device freely within the degrees of freedom of the first engagement interface and the second engagement interface, such as linearly along one or more of the engagement interfaces, diagonally in relation to the engagement interfaces or in a circular or spiral pattern.

The accessory device 400 comprises an interaction interface 403 for interacting with the electronic implant (not shown in Fig. 1). The interaction interface 403 may be one or more of a wireless charging interface 403A and a wireless communication interface 403B. The interaction interface 403 may be configured to obtain an interaction parameter associated with the interaction between the accessory device 400 and the electronic implant.

The one or more drive unit(s) may be controlled by one or more controller, such as a control circuitry, based on an obtained interaction parameter indicative of a quality of an interaction between the accessory device 400 and the electronic implant. The positioning device 100 may be configured to obtain the interaction parameter using the interaction interface 403, 403A, 403B of the accessory device 400. The positioning device 100 is configured to position, based on the obtained interaction parameter, the accessory device 400 in relation to the base 101, via the one or more interaction interfaces 102A, 102B. Upon the positioning device 100 being worn by a bearer of the electronic implant, the positioning device is configured to position the accessory device 400 in relation to the electronic implant, to improve the interaction, such as the quality of interaction, between the electronic implant and the accessory device 400.

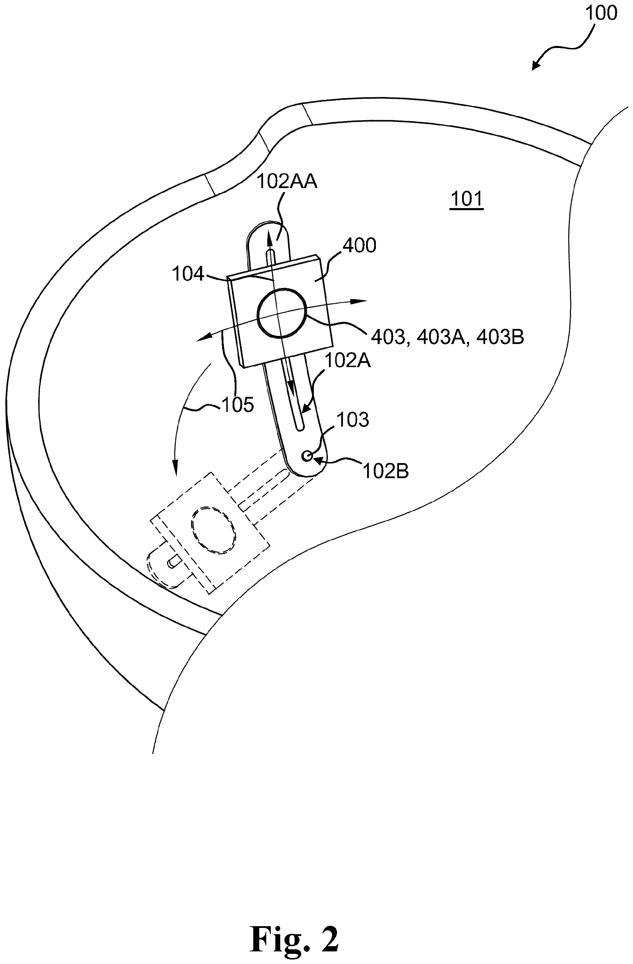

Fig. 2 shows an example positioning device 100 for positioning of the accessory device 400 in relation to an interaction interface of an electronic implant according to one or more examples disclosed herein. In the example positioning device 100 shown in Fig. 2, the first engagement interface 102A corresponds to the first interaction interface 102A of the example positioning device 100 of Fig. 1. The second engagement interface 102B of Fig. 2 however differs from the second engagement interface 102B of Fig. 1 in that it is configured to perform a rotational motion in relation to the base 101. The bracket 102AA is movably secured to the base 101 via a pivoting connection 103. The pivoting connection 103 is configured to enable the bracket 102AA to pivot, such as rotate, around a pivot point, such as a rotational axis, in relation to the base. The pivoting movement of the second engagement interface 102B, such as the pivoting movement of the bracket 102AA, may be performed by one of the one or more drive units (not shown in Fig. 2). By performing a rotational movement using the second engagement interface 102B and a translational motion using the first engagement interface 102A, the positioning device may be configured to move the accessory device in a spiral or meandering pattern over the positioning area of the positioning device, depending on whether the rotational movement rotates full circles or oscillates back and forth sweeping angles smaller than 360°. During the movement in the spiral pattern, the accessory device may obtain a plurality of sets of interaction parameters, where each set of interaction parameters is associated with, such as obtained at, a respective position in the positioning area. The example positioning device 100 may be configured to position the accessory device in the position associated with the set of interaction parameters being indicative of the highest interaction quality of the obtained sets of interaction parameters.

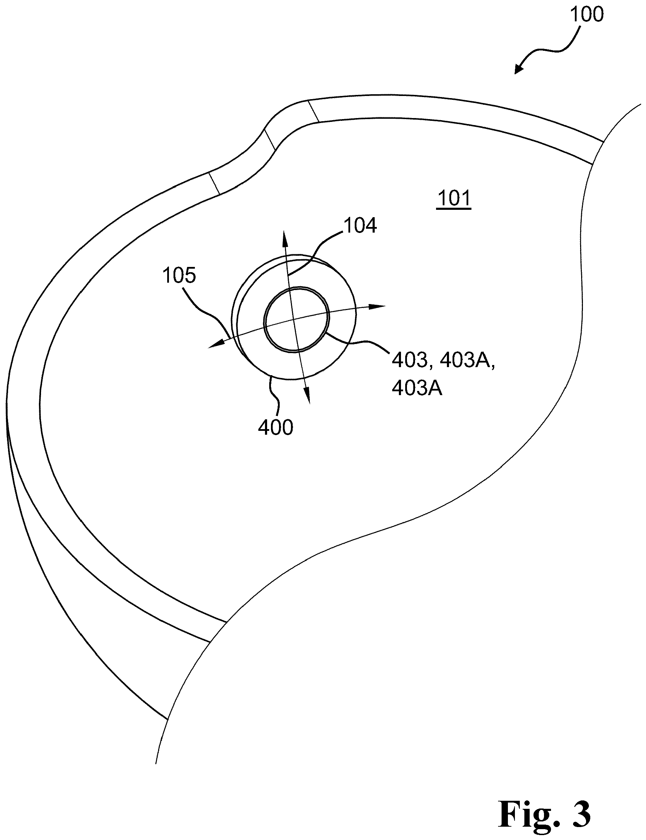

Fig. 3 shows an example positioning device 100 for positioning of the accessory device 400 in relation to an interaction interface of an electronic implant according to one or more examples disclosed herein. In the example positioning device 100 of Fig. 3, the accessory device 400 is configured to be freely movable over a surface of the base 101 by means of engagement interfaces . In the example shown in Fig. 3 the base 101 is the headpiece as shown in Figs. 1 and 2. The accessory device 400 may be configured to perform one or more of a first curved motion, a second curved motion, a curved motion, a translational motion, and a rotational motion. The base 101, such as the helmet, may be configured to provide space between the base 101 and the bearer of the electronic implant to allow the accessory device 400 to move between the base 101 and the bearer of the electronic implant. In one or more example positioning devices, the base 101, such as the headpiece, may comprise a set of spacers (not shown in Fig. 3) for spacing the base 101 from the bearer of the electronic implant. When the base 101 is shaped to match a contour of the body part of the bearer wherein the implant is located, it follows that the space between the base 101 and the bearer will be between the base 101 and the skin and that the space will at least substantially follow a contour of the relevant body part and therefore have a curved shape. When the positioning device moves within this curved space, it will perform curved motions. In one or more example positioning devices, the base 101 is shaped to at least substantially follow a contour of the body part of the bearer, such as the head, scalp, chest, or trunk region, whereby the space between the base 101 and the skin of the bearer will have a curved shape. In one or more example positioning devices, the headpiece comprises a headband for securing the headpiece to a head of a bearer of the electronic implant, and a set of spacers for spacing the headband from the base 101. The example positioning device 101 of Fig. 3 may be configured to position the accessory device 400 in a storage position when the accessory device 400 is not being actively used for interacting with the electronic implant. In the storage position, the accessory device 400 may be secured to the base 101 so that it is prevented from leaving the base 101, such as falling out of the headpiece. In one or more example methods, the base 101 may comprise a securing device (not shown in Fig. 3), into which the accessory device may be inserted, for example by means of the one or more engagement interfaces. The securing device may be an integral part of the base 101 or may be a separate part configured to be arranged on the base 101.

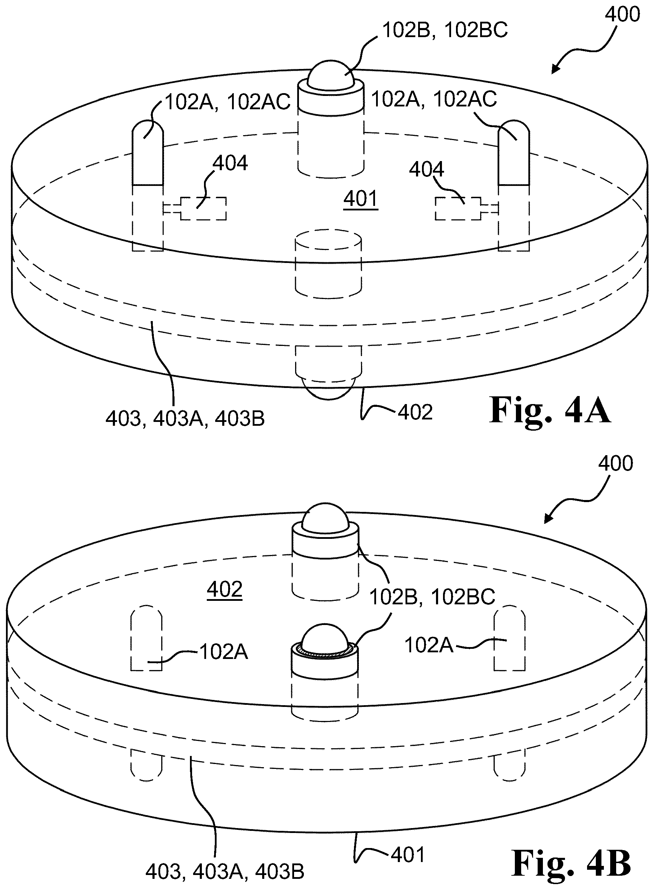

Figs. 4A-4B show detailed views of the example positioning device 100 of Fig. 3 configured to be freely movable on the base 101. Fig. 4A shows a perspective view of a base facing surface 401 of the accessory device 400. Fig. 4B shows a perspective view of a bearer facing surface 402 of the accessory device 400. The accessory device 400 may be configured to be sandwiched between the base 101 and the bearer of the electronic implant, so that the accessory device 400 abuts the base 101 on a first side, such as at the base facing surface 401, and the bearer of the electronic implant on an opposite second side, such as at a bearer facing side 402, and can move in the space between the base 101 and the bearer. In the example positioning device 400 of Fig. 4A, the first engagement interface 102A is a set of drive rollers 102AC, such as drive wheels. The set of drive rollers 102C may be arranged within the accessory device 400. The set of drive rollers 102C may protrude from a first surface 401 of the accessory device 400 the first surface 401 of the accessory device 400 being a base facing surface. The set of drive rollers 102AC may be connected to and may be drivable by the one or more drive units 404. The set of drive rollers 102AC are configured to engage with a bearer facing side of the base 101, for example using friction between the set of drive rollers 102AC and the base 101. The drive rollers in the set of drive rollers 102AC may be individually controlled, for example by the one or more controller, such as the control circuitry, of the positioning device 100, to perform one or more of the first and second motion, such as the rectilinear motion, the curved motion, the translational motion, and the rotational motion. In the example positioning device 100 of Figs. 4A-4B, the accessory device 400 further comprises one or more second engagement interfaces 102B, such as one or more spacers 102BC configured to space the surfaces of the accessory device 400 away from the base 101 and/or from the bearer of the implant and to stabilize the accessory device on the base 101. Spacing the surfaces of the accessory device 400 away from the base 101 and/or from the bearer of the implant can herein be seen as adjusting a distance between the accessory device 400 and the base 101 and/or the bearer of the implant. The spacers 102BC may be slidably arranged on the accessory device 400, such as in a housing of the accessory device 400. The spacers 102BC may comprise ball rollers configured to engage with the base 101 and/or the bearer of the electronic implant. The ball rollers can reduce the friction between the accessory device and the base 101 and/or the bearer of the electronic implant and may enable a smoother ride of the accessory device 400 over the surfaces of the base 101 and/or the bearer of the electronic implant. The spacers 102BC, such as the ball rollers, may be spring loaded. This allows the one or more spacers to follow the contours of the base and/or the bearer of the implant and compensate for irregularities in the contours of the base and/or the bearer.

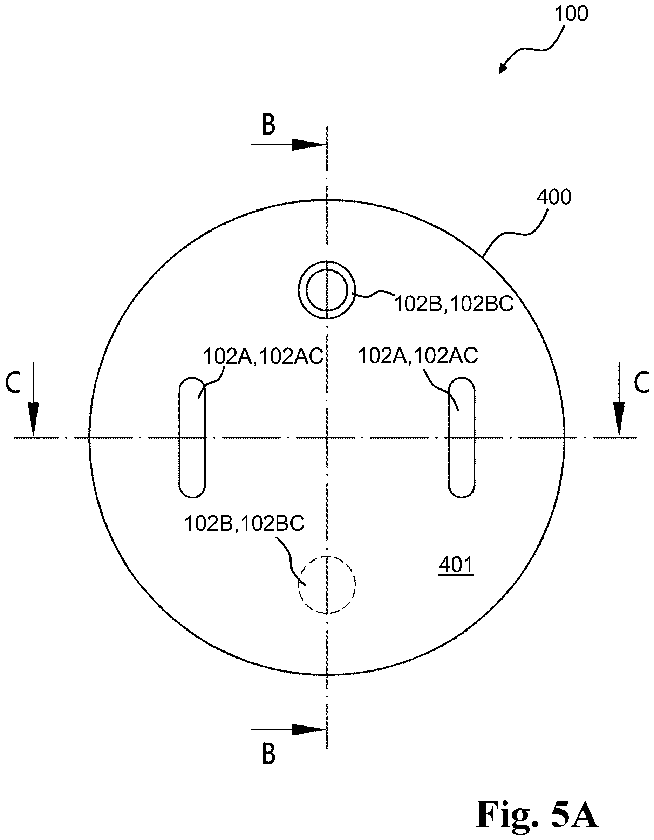

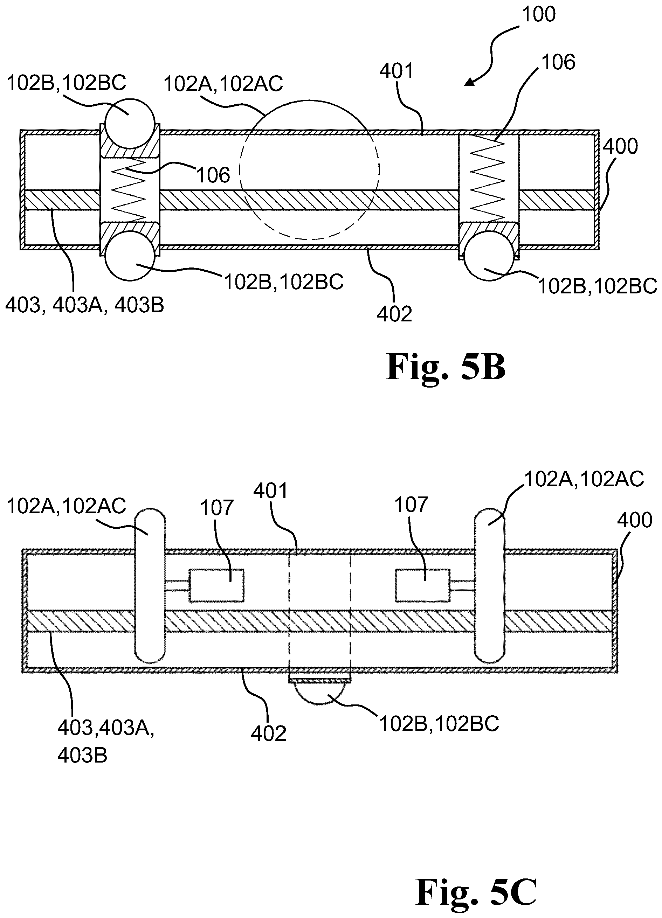

Figs. 5A-5C illustrate cross section views of the example positioning device 100 disclosed in Figs. 3-4B. Fig. 5A illustrates a schematic cut view of the example positioning device 100 seen in the direction of the base facing side 401 of the accessory device 400. Fig. 5B illustrates a schematic first cross-section view of the example positioning device along a cut-line B-B through the second engagement interfaces 102B, such as the spacers 102BC, as shown in Fig. 5A. Fig. 5C illustrates a schematic second cross-section view of the example positioning device along a cut-line C-C through the first engagement interfaces 102A, such as the one or more drive rollers 102AC, as shown in Fig. 5A.

As can be seen in Fig. 5B, the example positioning device 100 comprises the accessory device 400 and a plurality of spacers 102B arranged in the accessory device 400, such as within a housing of the accessory device 400. The plurality of spacers 102B may protrude through the base facing surface 401 and the bearer facing surface 402 of the accessory device 400. In the example positioning device 100 of Figs. 5A-5C, the spacers 102BC comprise ball rollers configured to engage with the base 101 and/or the bearer of the electronic implant. The spacers 102B, such as the ball rollers, are spring loaded within the accessory device 400 by means of one or more springs 106. The example positioning device 100 of Figs. 5A-5C further comprises the first engagement interface 102A, such as the one or more drive rollers 102AC arranged in the accessory device 400 and protruding through the base facing surface 401 of the accessory device 400. The example positioning device 100 of Figs. 5A-5C further comprises the interaction interface 403, 403A, 403B for interacting with the electronic implant. In the example positioning device 100 of Figs. 5A-5C, the interaction interface 403, 403A, 403B is arranged within accessory device 400 and can be configured to communicate with the electronic implant, such as an interaction interface of the electronic implant, through the surfaces of the accessory device 400.

As can be seen in the second cross section view of the positioning device in Fig. 5C, the example first engagement interface 102A, comprises a set of drive rollers 102AC, such as two drive rollers 102AC, arranged in parallel to each other in the accessory device 400, such as within a housing of the accessory device 400. In the example positioning device of Figs. 5A-5C, the drive rollers 102AC are driven by a respective drive unit 107, such as a respective electric motor, which enables the drive rollers 102AC to be individually driven. In one or more example positioning device, the drive rollers may be driven by a same drive unit S107 via a respective gear assembly (not shown in Fig. 5A-5C). Each gear assembly may comprise a gear box and/or a clutch that can be individually controlled, to enable the drive rollers 102AC to be individually driven.

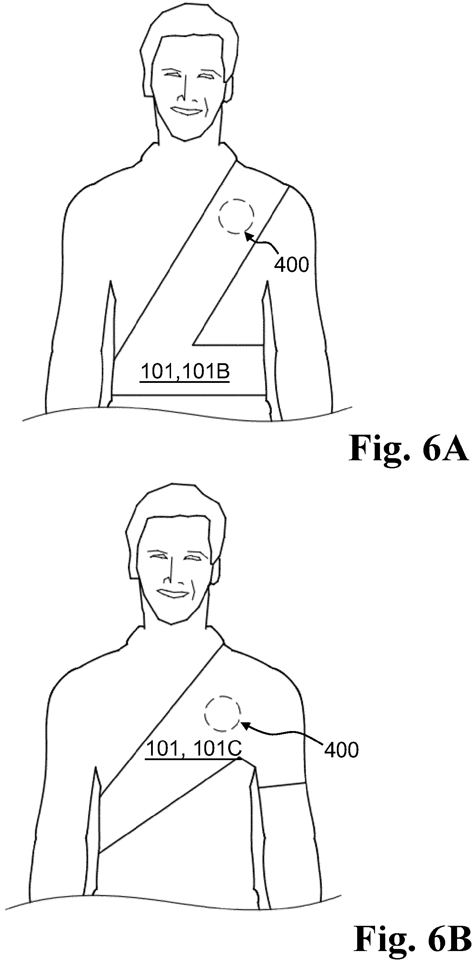

Figs. 6A-6B illustrate example bases 101B, 101C of the positioning device 100 according to one or more examples herein. The example bases 101 according to Figs. 6A-6B are configured to be worn on an upper body of the bearer of the electronic implant. The example bases 101B, 101C according to Figs. 6A-6B may be garments, such as elastic bandages, configured to cover an area of the body of the bearer where the electronic implant has been implanted. The first example base 101B of Fig. 6A may be configured as a vest being worn over a shoulder and around the waist of the bearer of the implant. The second example base 101C of Fig. 6B may be configured with a sleeve to be worn over an arm of the bearer of the electronic implant. The example bases 101B, 101C may be configured to be used with the example positioning device 100 of Figs. 3-5C. The example positioning device 100 of Figs. 3-5C may be arranged between the bearer of the electronic implant and the bases 101B, 101C, so that the drive rollers 102AC can engage the bases 101B, 101C to position the accessory device in relation to the electronic implant of the bearer. In this case, the electronic implant may be a pacemaker or any other electronic implant implanted in the upper body of the bearer.

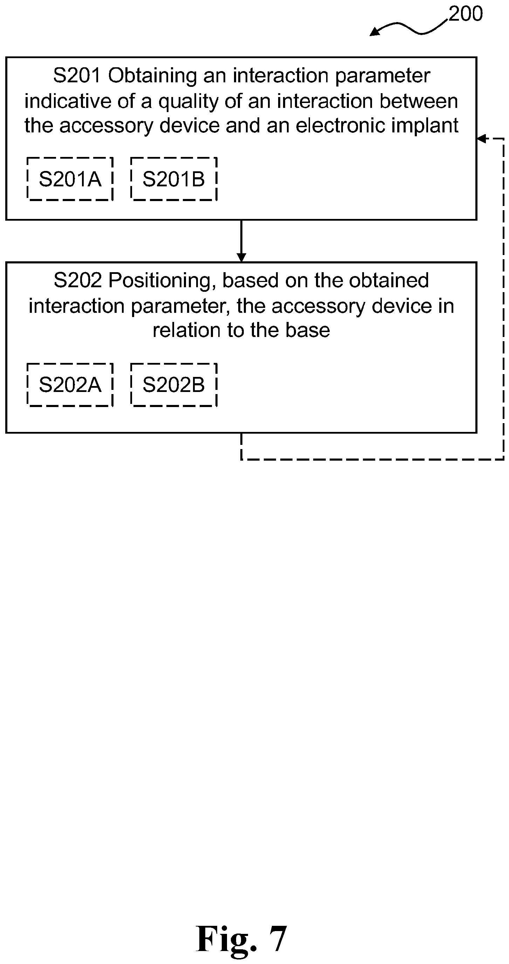

Fig. 7 discloses a flow diagram of an example method 200, performed by a positioning device according to the disclosure, for positioning an accessory device in relation to an interaction interface of an electronic implant. The positioning device may be the positioning device disclosed herein, such as the positioning device 100 of Figs. 1-5C. The positioning device may comprise a base, one or more engagement interfaces, one or more drive unit(s), the accessory device, and one or more controller for controlling the one or more drive unit(s). In one or more example methods, the one or more engagement interface(s) may be movably arranged in a translatory manner in relation to the base by means of one or more drive unit(s). The accessory device comprises an interaction interface for interacting with the electronic implant. The accessory device is arranged on one of the one or more positioning members.

The method comprises obtaining S201 an interaction parameter indicative of a quality of an interaction between the accessory device and the electronic implant.

In one or more example methods, obtaining S201 the interaction parameter comprises obtaining S201A a first set of interaction parameters associated with a first position of the accessory device in relation to the base. The first set of interaction parameters, may be a primary first set of interaction parameters, associated with a first position of the accessory device in relation to the base. The first position of the accessory device may be a current position, such as an initial position of the accessory device.

The first set of interaction parameters being associated with the first position can herein be seen as the first set of interaction parameters having been obtained when the accessory device is in the first position.

In one or more example methods, obtaining S201 the interaction parameter comprises obtaining S201B a second set, such as a primary second set of interaction parameters associated with a second position of the accessory device in relation to the base. The second set of interaction parameters being associated with the second position can herein be seen as the second set of interaction parameters having been obtained when the accessory device is in the second position. The second position may be different than the first position.

In one or more example methods, obtaining S201 the interaction parameter comprises moving the accessory device, for example using the engagement interfaces and/or the one or more drive units, to a plurality of positions and obtaining a set of interaction parameters at each respective position.

In one or more example methods, the method comprises determining a quality of each set of interaction parameters.

The method comprises positioning S202, based on the obtained interaction parameter, the accessory device in relation to the base. Positioning S202 may comprise positioning the accessory device in the position associated with the set of interaction parameters having the highest determined quality. In other words, the method may comprise positioning, based on the obtained first and second sets of interaction parameters, the accessory device in a position in which the interaction, such as the charging of the electronic implant and/or the communication with the electronic implant, is best.

In one or more example methods, positioning S202 the accessory device comprises, upon the second set of interaction parameters being indicative of a higher quality of interaction than the first set of interaction parameters, positioning S202A the accessory device in the second position. Upon the second set of interaction parameters associated with the primary second position having a higher quality than the set of interaction parameters associated with the primary first position, the method may comprise keeping the accessory device in the primary second position. The primary second position may then become a secondary first position for a new iteration of the method steps S201-S202.

In the new iteration of the methods steps S201-S202, obtaining S201B the set of interaction parameters may comprise moving the accessory device to a secondary second position and obtain a set of interaction parameters associated with the secondary second position. The secondary second position may be located on a same trajectory, such as on a first trajectory, as the move from the primary first position to the primary second position. As long as the quality of the set of interaction parameters in the second position, such as in a tertiary second position, a quaternary second position, etc., the positioning device may continue to move the accessory device along the same trajectory until the quality of the set of interaction parameters associated with the respective second position fails to improve. Thereafter, the positioning device may be configured to move the accessory device in a direction perpendicular to the direction of the previous trajectory, such as the first trajectory, to find an optimum position along the second trajectory, such as a position where the quality of the set of interaction parameters is optimal.

In one or more example methods, positioning S202 the accessory device comprises, upon the second set of interaction parameters being indicative of a lower quality of interaction than the first set of interaction parameters, positioning S202B the accessory device in the first position. In one or more example methods, such as when the primary first position is the same as the secondary first position, the secondary second position may be chosen in an opposite direction or a direction perpendicular to the direction of movement from the primary first position to the primary second position. In other words, for the new repetition of the method steps S201-S202B, the secondary second set of interaction parameters may be obtained in a direction different than the direction from the primary first position to the primary second position.

In one or more example methods, upon the accessory device being positioned in either the first position or the second position, this position is set, such as becomes, a new first position, such as a secondary first position. The secondary first position thus corresponds to one of the primary first position and the primary second position. The steps S201-S202 may then be repeated starting from the secondary first position and obtaining a secondary second set of interaction parameters associated with a secondary second position. The secondary second position is different from the primary first position and the primary second position.

In one or more example methods, the method steps S201-S202 may be repeated until no increase in quality of the interaction parameters is detected anymore or until the accessory device has covered an entire positioning area defined by the degrees of freedom of the first engagement interface and the second engagement interface and the positioning device has obtained sets of interaction parameters for the entire positioning area. The quality of the set of interaction parameters may be monitored, even after an optimal position has been determined, for example by performing steps S201A-S201B, and position optimization may be started by performing the method steps S202A-S202B, in case of significant deterioration of the monitored quality. Thereby any accidental movement, such as a slippage of the base and/or the accessory device in relation to the electronic implant and/or slippage of the accessory device in relation to the base.

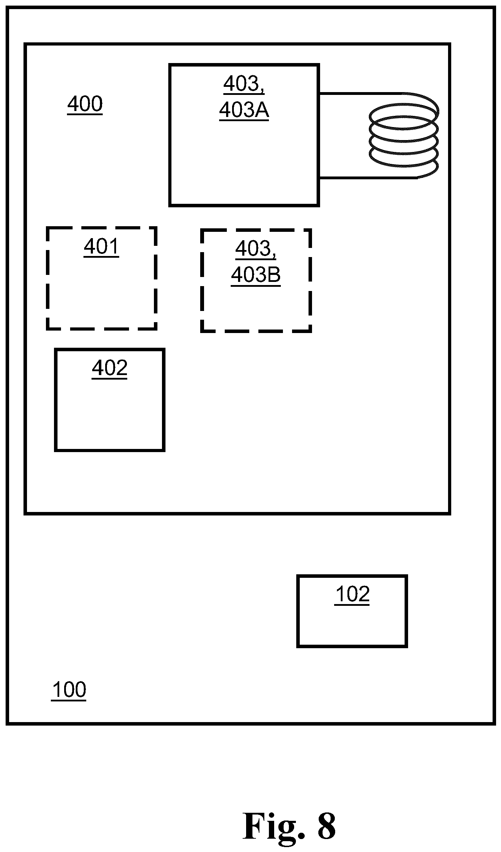

Fig. 8 shows a block diagram of an example positioning device 100 according to the disclosure. The positioning device 100 comprises the accessory device 400, processor circuitry 402, an interaction interface 403 for interacting with the electronic implant, such as a charging interface 403A and/or a communication interface 403B, and one or more engagement interfaces 102 for positioning the accessory device in relation to a base of the positioning device 100. In one or more examples, the positioning device 100, such as the accessory device 400, comprises a memory circuitry 401. The positioning device is configured to position the accessory device 400 in relation to an interaction interface of an electronic implant. The wireless charging interface 403A may be an inductive charging coil, such as an emitting charging coil, or a CPT coupling coupler, such as an emitting CPT coupling coupler. The positioning device 100 may be configured to perform any of the methods disclosed in Fig. 7. In other words, the positioning device 100 may be configured to obtain an interaction parameter indicative of a quality of an interaction between the accessory device 400 and the electronic implant. The positioning device 100 may be configured to position, based on the obtained interaction parameter, the accessory device in relation to the base of the positioning device, and when the positioning device is worn by a bearer of the electronic implant, in relation to the electronic implant. The components 102B, 400, 401, 402, 403, 403A, 403B of the positioning device 100 may be arranged in one or more housings, such as may be arranged in the same housing or may be distributed into different housings.

The positioning device 100, such as the accessory device 400, is configured to interact with the electronic implant, such as the electronic implant disclosed herein, using the interaction interface 403, such as the charging interface 403A and/or the communication interface 403B.

In one or more examples, the accessory device 400 may be configured to generate a magnetic field (for example using the second wireless charging interface 403A, such as the inductive charging coil or the CPT coupling coupler), which can be detected by a first wireless charging interface, such as an inductive charging coil or a CPT coupling coupler, in the electronic implant.

The accessory device 400 is configured to obtain, such as determine and/or receive (for example using the communication interface 403B), from the electronic implant, a signal indicative of set of interaction parameters, such as indicative of a communication parameter and/or a charging parameter, in one or more positions of the accessory device 400 in relation to the base of the positioning device and/or the interaction interface of the electronic implant.

The accessory device 400 may be configured to determine, based on the obtained set of interaction parameters, a quality of the interaction between the accessory device and the electronic implant. In one or more examples, the accessory device 400 is configured to determine, based on the charging parameter, a quality of charging. In one or more examples, the accessory device 400 is configured to determine, based on the communication parameter, a quality of communication.

The accessory device 400 may be configured to position the accessory device 400 (for example using the one or more engagement interfaces 102), based on the determined quality of the interaction between the accessory device and the electronic implant for a plurality of positions. The positioning device 100 may be configured to position the accessory device in a position associated with the set of interaction parameters having the highest quality, such as a higher quality than the quality that the other obtained sets of interaction parameters associated with other positions of the accessory device have.

The communication interface 403 may be configured for wireless communications via a wireless communication system, such as via one or more of Bluetooth, Wi-Fi, Near-Field Communication (NFC), Medical Implant Communication Service (MICS), and Industrial-Scientific-Medical (ISM), or a mobile communication system, such as a 3^{rd} Generation Partnership Program (3GPP) system.

The positioning device 100, such as the accessory device 400, is configured to send, for example, via the wireless interface 403, to a user equipment, information indicative of one or more interaction parameter.

Processor circuitry 402 is optionally configured to perform any of the operations disclosed in Fig. 7 (such as any one or more of S201, S201A, S201B, S202, S202A, S202B). The operations of the positioning device 100 may be embodied in the form of executable logic routines (for example, lines of code, software programs, etc.) that are stored on a non-transitory computer readable medium (for example, memory circuitry 401) and are executed by processor circuitry 402.

Furthermore, the operations of the positioning device 100 may be considered a method that the positioning device 100 is configured to carry out. Also, while the described functions and operations may be implemented in software, such functionality may also be carried out via dedicated hardware or firmware, or some combination of hardware, firmware and/or software.

Memory circuitry 401 may be one or more of a buffer, a flash memory, a hard drive, a removable media, a volatile memory, a non-volatile memory, a random access memory (RAM), or other suitable device. In a typical arrangement, memory circuitry 401 may include a non-volatile memory for long term data storage and a volatile memory that functions as system memory for processor circuitry 402. Memory circuitry 401 may exchange data with processor circuitry 402 over a data bus. Control lines and an address bus between memory circuitry 401 and processor circuitry 402 also may be present (not shown in Fig. 8). Memory circuitry 401 is considered a non-transitory computer readable medium.

Memory circuitry 401 may be configured to store information (such as information indicative of the interaction parameter, such as the communication parameter and/or the charging parameter, and/or the charge level) in a part of the memory.

Examples of methods and products (positioning device and method for positioning an accessory device) according to the disclosure are set out in the following items:
Item 1. A positioning device (100) for positioning an accessory device (400) in relation to an interaction interface of an electronic implant, the positioning device (100) being configured to be worn by a bearer of the electronic implant,
   the positioning device (100) comprising a base (101), one or more engagement interfaces (102), one or more drive unit(s) (107), the accessory device (400), and one or more controller for controlling the one or more drive unit(s) (107), the one or more engagement interfaces (102) comprising a first engagement interface (102A) movably arranged in relation to the base (101),
   the accessory device (400) comprising an interaction interface (403) for interacting with the electronic implant, wherein the accessory device (400) is arranged on the first engagement interface (102A),
   wherein the positioning device (100) is configured to obtain an interaction parameter indicative of a quality of an interaction between the accessory device and the electronic implant, and
   wherein the positioning device (100) is configured to position, based on the obtained interaction parameter, the accessory device (400) in relation to the base (101).
Item 2. The positioning device (100) according to Item 1, wherein the first engagement interface (102A) is configured to perform a first rectilinear motion in relation to the base (101).
Item 3. The positioning device (100) according to Item 1 or 2, wherein the positioning device (100) comprises a second engagement interface (102B), the accessory device (400) being movably arranged in a primary direction via the first engagement interface (102A) and the first engagement interface (102A) being movably arranged in a secondary direction via the second engagement interface (102B).
Item 4. The positioning device (100) according to Item 3, wherein the primary direction and the secondary direction are substantially perpendicular to each other.
Item 5. The positioning device (100) according to any of the Items 3 to 4, wherein the second engagement interface (102B) is configured to perform a rotational motion in relation to the base (101).
Item 6. The positioning device (100) according to any of the Items 3 to 4, wherein the second engagement interface (102B) is configured to perform a second rectilinear motion in relation to the base (101), wherein the second rectilinear motion is different to the first rectilinear motion.
Item 7. The positioning device (100) according to any one of the previous Items, wherein the base (101) has a first side configured to face the bearer of the electronic implant and a second side being configured to face away from the bearer of the electronic implant, wherein the first engagement interface (102A) and the accessory device (400) are arranged on a bearer facing side of the base (101).
Item 8. The positioning device (100) according to any of the previous Items, wherein the interaction interface (403) is a wireless charging interface (403A), and the interaction parameter is indicative of a quality of charging.
Item 9. The positioning device (100) according to Item 8, wherein the wireless charging interface (403A) is a charging coil.
Item 10. The positioning device (100) according to any of the previous Items, wherein the interaction interface (403) is a wireless communication interface (403B), and the interaction parameter is indicative of a quality of communication.
Item 11. The positioning device (100) according to any one of the previous Items, wherein the positioning device (100) is configured to:
   - obtain a first set of interaction parameters associated with a first position of the accessory device (400) in relation to the base (101), wherein the first position of the accessory device is a current position,
   - obtain a second set of interaction parameters associated with a second position of the accessory device (400) in relation to the base (101),
   - upon the second set of interaction parameters being indicative of a higher quality of interaction than the first set of interaction parameters, position, using one or more of the engagement interfaces, the accessory device (400) in the second position, and
   - upon the second set of interaction parameters being indicative of a lower quality of interaction than the first set of interaction parameters, position the accessory device (400) in the first position.
Item 12. The positioning device (100) according to any of the previous Items, wherein the base (101) is a headpiece.
Item 13. The positioning device according to Item 12, wherein the headpiece comprises a headband (101A) for securing the headpiece to a head of a bearer of the electronic implant, and a set of spacers for spacing the headband from the base (101).
Item 14. The positioning device (100) according to any of the previous Items, wherein the base (101) is a piece of garment.
Item 15. The positioning device according to any one of the previous Items, wherein the base (101) is a housing.
Item 16. A method, performed in a positioning device, for positioning an accessory device in relation to an interaction interface of an electronic implant, the positioning device comprising a base, one or more engagement interfaces, one or more drive unit(s), the accessory device, and one or more controller for controlling the one or more drive unit(s), the one or more engagement interface(s) being movably arranged in relation to the base by means of the one or more drive unit(s), the accessory device comprising an interaction interface for interacting with the electronic implant, wherein the accessory device is arranged on one of the one or more engagement interfaces, the method comprising:
   - obtaining (S201) an interaction parameter indicative of a quality of an interaction between the accessory device and the electronic implant, and
   - positioning (S202), based on the obtained interaction parameter, the accessory device in relation to the base.
Item 17. The method according to Item 16, wherein obtaining (S202) the interaction parameter comprises:
   - obtaining (S201A) a first set of interaction parameters associated with a first position of the accessory device in relation to the base, wherein the first position of the accessory device is a current position,
   - obtaining (S201B) a second set of interaction parameters associated with a second position of the accessory device in relation to the base.
Item 18. The method according to Item 17, wherein positioning (S202) the accessory device comprises:
   - upon the second set of interaction parameters being indicative of a higher quality of interaction than the first set of interaction parameters, positioning (S202A) the accessory device in the second position.
Item 19. The method according to Item 17 or 18, wherein positioning (S202) the accessory device comprises:
   - upon the second set of interaction parameters being indicative of a lower quality of interaction than the first set of interaction parameters, positioning (S202B) the accessory device in the first position.

The use of the terms "first", "second", "third" and "fourth", "primary", "secondary", "tertiary" etc. does not imply any particular order but are included to identify individual elements. Moreover, the use of the terms "first", "second", "third" and "fourth", "primary", "secondary", "tertiary" etc. does not denote any order or importance, but rather the terms "first", "second", "third" and "fourth", "primary", "secondary", "tertiary" etc. are used to distinguish one element from another. Note that the words "first", "second", "third" and "fourth", "primary", "secondary", "tertiary" etc. are used here and elsewhere for labelling purposes only and are not intended to denote any specific spatial or temporal ordering. Furthermore, the labelling of a first element does not imply the presence of a second element and vice versa.

It may be appreciated that Figures 1-8 comprise some circuitries or operations which are illustrated with a solid line and some circuitries, components, features, or operations which are illustrated with a dashed line. Circuitries or operations which are comprised in a solid line are circuitries, components, features or operations which are comprised in the broadest example. Circuitries, components, features, or operations which are comprised in a dashed line are examples which may be comprised in, or a part of, or are further circuitries, components, features, or operations which may be taken in addition to circuitries, components, features, or operations of the solid line examples. It should be appreciated that these operations need not be performed in order presented. Furthermore, it should be appreciated that not all of the operations need to be performed. The example operations may be performed in any order and in any combination. It should be appreciated that these operations need not be performed in order presented. Circuitries, components, features, or operations which are comprised in a dashed line may be considered optional.

Other operations that are not described herein can be incorporated in the example operations. For example, one or more additional operations can be performed before, after, simultaneously, or between any of the described operations.

Certain features discussed above as separate implementations can also be implemented in combination as a single implementation. Conversely, features described as a single implementation can also be implemented in multiple implementations separately or in any suitable sub-combination. Moreover, although features may be described above as acting in certain combinations, one or more features from a claimed combination can, in some cases, be excised from the combination, and the combination may be claimed as any sub-combination or variation of any sub-combination

It is to be noted that the word "comprising" does not necessarily exclude the presence of other elements or steps than those listed.

It is to be noted that the words "a" or "an" preceding an element do not exclude the presence of a plurality of such elements.

It should further be noted that any reference signs do not limit the scope of the claims, that the examples may be implemented at least in part by means of both hardware and software, and that several "means", "units" or "devices" may be represented by the same item of hardware.

Language of degree used herein, such as the terms "approximately," "about," "generally," and "substantially" as used herein represent a value, amount, or characteristic close to the stated value, amount, or characteristic that still performs a desired function or achieves a desired result. For example, the terms "approximately", "about", "generally," and "substantially" may refer to an amount that is within less than or equal to 10% of, within less than or equal to 5% of, within less than or equal to 1% of, within less than or equal to 0.1% of, and within less than or equal to 0.01% of the stated amount. If the stated amount is 0 (e.g., none, having no), the above recited ranges can be specific ranges, and not within a particular % of the value. For example, within less than or equal to 10 wt./vol. % of, within less than or equal to 5 wt./vol. % of, within less than or equal to 1 wt./vol. % of, within less than or equal to 0.1 wt./vol. % of, and within less than or equal to 0.01 wt./vol. % of the stated amount.

The various example methods, devices, and systems described herein are described in the general context of method steps or processes, which may be implemented in one aspect by a computer program product, embodied in a computer-readable medium, including computer-executable instructions, such as program code, executed by computers in networked environments. A computer-readable medium may include removable and non-removable storage devices including, but not limited to, Read Only Memory (ROM), Random Access Memory (RAM), compact discs (CDs), digital versatile discs (DVD), etc. Generally, program circuitries may include routines, programs, objects, components, data structures, etc. that perform specified tasks or implement specific abstract data types. Computer-executable instructions, associated data structures, and program circuitries represent examples of program code for executing steps of the methods disclosed herein. The particular sequence of such executable instructions or associated data structures represents examples of corresponding acts for implementing the functions described in such steps or processes.

Although features have been shown and described, it will be understood that they are not intended to limit the claimed disclosure, and it will be made obvious to those skilled in the art that various changes and modifications may be made without departing from the scope of the claimed disclosure. The specification and drawings are, accordingly, to be regarded in an illustrative rather than restrictive sense. The claimed disclosure is intended to cover all alternatives, modifications, and equivalents.

## Claims

1. A positioning device (100) for positioning an accessory device (400) in relation to an interaction interface of an electronic implant, the positioning device (100) being configured to be worn by a bearer of the electronic implant,
the positioning device (100) comprising a base (101), one or more engagement interfaces (102), one or more drive unit(s) (107), the accessory device (400), and one or more controller for controlling the one or more drive unit(s) (107), the one or more engagement interfaces (102) comprising a first engagement interface (102A) movably arranged in relation to the base (101), and the accessory device (400) being arranged on the first engagement interface (102A),
the accessory device (400) comprising an interaction interface (403) for interacting with the electronic implant,
wherein the positioning device (100) is configured to move the accessory device (400) in a curved motion,
wherein the positioning device (100) is configured to obtain an interaction parameter indicative of a quality of an interaction between the accessory device and the electronic implant, and
wherein the positioning device (100) is configured to position, based on the obtained interaction parameter, the accessory device (400) in relation to the base (101).

2. The positioning device (100) according to claim 1, wherein the first engagement interface (102A) is configured to perform a first curved motion.

3. The positioning device (100) according to any one of the previous claims, wherein the first engagement interface (102A) comprises a bracket (102AA) and a first sliding connector (102AB), and wherein the bracket and/or the first sliding connector has a curved shape that provides the curved motion when the accessory device (400) is moved.

4. The positioning device (100) according to any one of the previous claims, wherein the positioning device (100) comprises a second engagement interface (102B), the accessory device (400) being movably arranged in a primary direction via the first engagement interface (102A) and the first engagement interface (102A) being movably arranged in a secondary direction via the second engagement interface (102B).

5. The positioning device (100) according to claim 4, wherein the second engagement interface (102B) is configured to perform a rotational motion in relation to the base (101).

6. The positioning device (100) according to claim 4, wherein the second engagement interface (102B) is configured to perform a second curved motion, wherein the second curved motion is different to the first curved motion.

7. The positioning device (100) according to any one of the previous claims, wherein the base (101) has a first side configured to face the bearer of the electronic implant and a second side being configured to face away from the bearer of the electronic implant, wherein the first engagement interface (102A) and the accessory device (400) are arranged on a bearer facing side of the base (101).

8. The positioning device (100) according to any of the previous claims, wherein the interaction interface (403) is a wireless charging interface (403A), and the interaction parameter is indicative of a quality of charging.

9. The positioning device (100) according to any one of the previous claims, wherein the positioning device (100) is configured to:
- obtain a first set of interaction parameters associated with a first position of the accessory device (400) in relation to the base (101), wherein the first position of the accessory device is a current position,
- obtain a second set of interaction parameters associated with a second position of the accessory device (400) in relation to the base (101),
- upon the second set of interaction parameters being indicative of a higher quality of interaction than the first set of interaction parameters, position, using one or more of the engagement interfaces, the accessory device (400) in the second position, and
- upon the second set of interaction parameters being indicative of a lower quality of interaction than the first set of interaction parameters, position the accessory device (400) in the first position.

10. The positioning device (100) according to any of the previous claims, wherein the base (101) is a headpiece.

11. The positioning device according to claim 10, wherein the headpiece comprises a headband (101A) for securing the headpiece to a head of a bearer of the electronic implant, and a set of spacers for spacing the headband from the base (101).

12. A method, performed in a positioning device, for positioning an accessory device in relation to an interaction interface of an electronic implant, the positioning device comprising a base, one or more engagement interfaces, one or more drive unit(s), the accessory device, and one or more controller for controlling the one or more drive unit(s), the one or more engagement interface(s) being movably arranged in a translatory manner in relation to the base by means of the one or more drive unit(s), and the accessory device is arranged on one of the one or more positioning members, wherein the positioning device (100) is configured to move the accessory device (400) in a curved motion, and wherein the accessory device comprises an interaction interface for interacting with the electronic implant, the method comprising:
- obtaining (S201) an interaction parameter indicative of a quality of an interaction between the accessory device and the electronic implant, and
- positioning (S202), based on the obtained interaction parameter, the accessory device in relation to the base by moving the accessory device (400) in a curved motion.

13. The method according to claim 12, wherein obtaining (S201) the interaction parameter comprises:
- obtaining (S201A) a first set of interaction parameters associated with a first position of the accessory device in relation to the base, wherein the first position of the accessory device is a current position,
- obtaining (S201B) a second set of interaction parameters associated with a second position of the accessory device in relation to the base.

14. The method according to claim 13, wherein positioning (S202) the accessory device comprises:
- upon the second set of interaction parameters being indicative of a higher quality of interaction than the first set of interaction parameters, positioning (S202A) the accessory device in the second position.

15. The method according to claim 13 or 14, wherein positioning (S202) the accessory device comprises:
- upon the second set of interaction parameters being indicative of a lower quality of interaction than the first set of interaction parameters, positioning (S202B) the accessory device in the first position.
